## Europäisches Patentamt

## European Patent Office

## Office européen des brevets

(19)

(11) Veröffentlichungsnummer: **0 004 896**
**B1**

(12) **EUROPÄISCHE PATENTSCHRIFT**

(45) Veröffentlichungstag der Patentschrift: **20.05.81**

(51) Int. Cl.³: **C 07 D 417/12**

(21) Anmeldenummer: **79100983.0**

(22) Anmeldetag: **31.03.79**

(54) Verfahren zur Herstellung von Benzthiazolylsulfensäuremorpholid.

(30) Priorität: **13.04.78 DE 2815964**

(43) Veröffentlichungstag der Anmeldung:
**31.10.79 Patentblatt 79/22**

(45) Bekanntmachung des Hinweises auf die Patenterteilung:
**20.05.81 Patentblatt 81/20**

(84) Benannte Vertragsstaaten:
**BE DE FR GB IT NL**

(56) Entgegenhaltungen:
**DE - B - 1 170 412**
**US - A - 2 271 834**
**US - A - 2 782 202**
**US - A - 3 144 652**
**US - A - 3 178 428**

(73) Patentinhaber: **BAYER AG**
**Zentralbereich Patente, Marken und Lizenzen**
**D-5090 Leverkusen 1, Bayerwerk (DE)**

(72) Erfinder: **Hüllstrung, Dieter, Dr.**
**Friedrich-Bayer-Strasse 11**
**D-5090 Leverkusen 1 (DE)**
Erfinder: **Wicke, Manfred, Dr.**
**Paul-Humburg-Strasse 66**
**D-5000 Köln 60 (DE)**
Erfinder: **Scherhag, Bernhard, Dr.**
**Elisabeth-Langgässer-Strasse 2**
**D-5090 Leverkusen 1 (DE)**

Courier Press, Leamington Spa, England.

# Verfahren zur Herstellung von Benzthiazolylsulfensäuremorpholid

Die vorliegende Erfindung betrifft ein Verfahren zur Herstellung von 2-Benzthiazolylsulfensäuremorpholid (nachfolgend als MBS bezeichnet), bei dem MBS als Schmelze aus einem wäßrigen Reaktionsgemisch abgetrennt und in hoher Reinheit und Lagerstabilität erhalten wird.

Das wäßrige Reaktionsgemisch fällt an bei der Synthese von MBS aus 2-Benzthiazolyldisulfid, Mercaptobenzthiazol oder dessen Natriumsalz und einem mehrfachen molaren Überschuß von Morpholin durch Einwirkung eines Oxydationsmittels wie NaOCl, $Cl_2$ oder $H_2O_2$. MBS stellt einen wichtigen Vulkanisationsbeschleuniger aus der Klasse der Sulfenamide dar.

Es ist bekannt, die Synthese von Benzthiazolylsulfenamiden in einem organischen Lösungsmittel durchzuführen. Das gewünschte Endprodukt fällt hierbei als Lösung im organischen Lösungsmittel an und wird entweder durch Wasserzugabe ausgefällt oder durch Abdampfen des Lösungsmittels als Schmelze gewonnen (US—Patenschrift 2 782 202).

Es ist ferner bekannt (US—Patentschrift 3 178 428), MBS in einem wäßrigen Gemisch herzustellen, in welchem das Sulfenamid unlöslich ist. Man kann hierbei die Temperatur so hoch wählen, daß das MBS direkt als Schmelze anfällt. In diesem Falle darf jedoch die molare Menge des eingesetzten Morpholins das 1,5 fache der molaren Menge des vorgelegten Benzthiazolderivats nicht überschreiten, um eine vorzeitige Zersetzung des gebildeten MBS, d.h. noch während des Reaktionsablaufs, zu vermeiden. Der geringe Überschuß an Morpholin von 50 Mol-% hat andererseits eine Reaktionszeit von mehreren Stunden zur Folge.

Will man die Reaktionszeit dagegen kurz halten, so muß die Reaktion im wäßrigen Gemisch gewöhnlich mit einem molaren Morpholinüberschuß von 200% oder mehr durchgeführt werden. Um bei einem derart großen Morpholinüberschuß eine Zersetzung des MBS bereits beim der Herstellung zu vermeiden, muß die Reaktionstemperatur wesentlich unter dem Schmelzpunkt des MBS (ca. 82—85°C) gehalten werden. Gewöhnlich wird die Reaktion daher bei 30—60°C durchgeführt.

Es ist bekannt daß erhebliche Nachteile damit verbunden sind, das aus einem Reaktionsgemisch mit großem Morpholinüberschuß hergestellte MBS in den geschmolzenen Zustand zu überführen und dort zu halten, da MBS eine relative instabile Substanz ist, die sich bei erhöhter Temperatur schnell zersetzt. Es ist ferner bekannt, daß die Zersetzung der Sulfenamide umso schneller abläuft, je höher die Konzentration an freiem Amin ist.

Man ist daher bis jetzt gezwungen, die wäßrigen Reaktionsgemische aus der Herstellung von MBS bei Verwendung eines großen molaren Überschusses an Morpholin als Suspension bei Raumtemperatur aufzuarbeiten, d.h., indem man z.B. das kristalline unlösliche MBS aus dem Reaktionsgemisch abfiltriert, durch intensives Auswaschen mit großen Mengen an Wasser von der Mutterlauge befreit und anschließend trocknet. Diese Aufarbeitungsschritte, wie auch die Rückgewinnung des Morpholins aus den Waschwässern, sind aber zeitraubende und teure Operationen.

Bei der Herstellung von MBS mit einem großen Morpholinüberschuß wird eine Suspension von festem MBS in einem Gemisch von Wasser und Morpholin erhalten, in dem noch organische Nebenprodukte und gegebenenfalls bei der Reaktion gebildete anorganische Salze gelöst sind. Eine solche Suspension enthält pro Mol MBS mehr als 0,5 Mol, i.a. 1,5 bis 2 Mol Morpholin.

Es wurde nun gefunden, daß aus solchen Suspensionen MBS in Form einer Schmelze von hoher Qualität und Stabilität erhalten wird, wenn man die Suspension unter Aufschmelzen des kristallinen MBS erwärmt, die entstandene Schmelzphase schnell von der wäßrigen Phase abtrennt, und anschließend die Schmelzphase von den noch vorhandenen flüchtigen Anteilen wie Wasser und Morpholin befreit. Diese Verfahrensschritte werden unmittelbar aufeinanderfolgend in einer Gesamtzeit von nicht mehr als 15 Minuten durchgeführt.

Gegenstand der Erfindung ist somit ein Verfahren zur Herstellung von 2-Benzthiazolylsulfensäuremorpholid durch Umsetzung von 2-Benzthiazolyl-disulfid, Mercaptobenzthiazol oder dessen Natriumsalz mit Morpholin unter Einwirkung eines Oxydationsmittels in wäßrigem Medium, dadurch gekennzeichnet, daß aus der erhaltenen Suspension die pro Mol 2-Benzthiazolylsulfensäuremorpholid mehr als 0,5 Mol Morpholin enthält das Reaktionsprodukt in geschmolzenem Zustand abgetrennt wird durch

a) Erhitzen der Suspension unter Aufschmelzen des festen Reaktionsprodukts,

b) anschließende schnelle Abtrennung der Schmelzphase von der wäßrigen Phase, und

c) Entfernung der in der Schmelze noch vorhandenen flüchtigen Antiele,

wobei die Summe der Verweilzeiten zwischen Beginn und Ende der drei Verfahrensschritte a) bis c) nicht mehr als 15 Minuten beträgt.

Überraschenderweise wird mit diesem Verfahren trotz des ursprünglich großen Morpholinüberschusses eine lagerstabile Schmelze von MBS erhalten, in der die sonst auftretende schnelle Zersetzung von MBS nicht mehr beobachtet wird.

Das erfindungsgemäße Verfahren ist in gleicher Weise zur Herstellung und Abtrennung von analogen Reaktionsprodukten, insbesondere von 2 - Benzthiazolylsulfensäure - 2,6 - dimethylmorpholid geeignet.

Zur Durchführung der Teilschritte des erfindungsgemäßen Verfahrens können an sich bekannte Methoden angewendet werden. So kann die Erwärmung der Suspension und ihre Überführung in ein Gemisch aus wäßriger Phase und Schmelzphase durch direkte Energiezufuhr oder indirekt, z.B. in einem Wärmeaustauscher, durchgeführt werden. Die daran anschließende Abtrennung der Schmelzphase kann durch Einwirkung von Fliehkraft, z.B. in einem Separator, oder durch Schwerkraft, z.B. in einer Trennflasche erfolgen. Die abschließende Befreiung der Schmelzphase von flüchtigen Anteilen wie Wasser und Morpholin wird zweckmäßigerweise durch Kurzzeitverdampfung erriecht, vorzugsweise unter Vakuum, z.B. in einem Dünnschichtverdampfer.

Bei dem Teilschritt der Ausdampfung der Schmelzphase hat es sich als zusätzlicher Vorteil erwiesen, dem ablaufenden Schmelzfilm einen Gasstrom, z.B. Stickstoff, Luft und/oder Wasserdampft, entgegenzuschicken.

Durch die beschriebene Verfahrensweise werden die zeitraubenden Schritte der Filtration, Wäsche und Trocknung des in Suspension in Gegenwart eines hohen Morpholinüberschusses erzeugten MBS ebenso vermieden wie die kostspielige Rückgewinnung des Morpholins aus den Waschwässern.

Die Schmelze des MBS, die nach Teilschritt c) anfällt, kann in bekannter Weise durch Abkühlen in den festen kristallinen Zustand überführt und das MBS in eine handelsübliche Form gebracht werden. Die Erfindung wird durch die nachstehenden Beispiele erläutert. Die %-Angaben sind Gew.-%.

### Beispiel 1

150 kg eines bei der Synthese von MBS anfallenden Reaktionsgemisches mit der Zusammensetzung 20% MBS, 20% Morpholin, 10% Kochsalz, 49% Wasser und 1% Nebenprodukt und einer Temperatur von ca. 30°C werden durch einen dampfbeheizten Rohrwärmetauscher gepumpt. Die Verweilzeit beim Aufheizen beträgt 1,6 Minuten. Aus dem Rohrwärmetauscher wird das Gemisch mit einer Temperatur von ca. 85°C dem vorderen Ende einer mit 3° geneigten Trennflasche zugeführt. Während hier die wäßrige Phase an der höchsten Stelle des hinteren Endes der Trennflasche in einen Sammelbehälter abfließt, sinken die Schmelztropfen zu Boden und fließen als Schicht dem tiefsten Punkt der Flasche zu, wo sie durch ein Bodenablaßventil kontinuierlich entnommen werden. Die Standhöhe der Schmelzphase wird mit Hilfe des Ventils so eingestellt, daß eine Verweilzeit von 4 Minuten nicht überschritten wird.

An das Bodenablaßventil der Trennflasche ist ein kurzes geneigtes Rohrstück angeschlossen, das am Zulauf eines handelsüblichen Dünnschichtverdampfers von 0,2 m² Heizfläche mündet. Der Dünnschichtverdampfer wird bei 30 Torr und 120°C betreiben. An seinem unteren Ende werden ca. 2 kg/h Wasserdampf gleicher Temperatur eingeblasen. Die Verweilzeit im Dünnschichtverdampfer beträgt weniger als 1 Minute. Aus dem Sumpf des Dünnschichtverdampfers werden ca. 30 kg/h einer Schmelze von MBS abgezogen, deren Restgehalt an Morpholin ca. 0,2 Gew.-% beträgt und die eine ausgezeichnete Lagerstabilität besitzt. Die Schmelze kann durch Abkühlen auf einer Schabewalze in die handelsübliche Schuppenform überführt werden.

### Beispiel 2

138 kg/h eines Reaktionsgemisches aus der Synthese von MBS mit der Zusammensetzung 21,7% MBS, 21,7% Morpholin, 1,1% organische Nebenprodukte und 55,5% Wasser werden durch ein wärmeisoliertes Rohr geleitet. Kurz hinter dem Eingang des Rohres wird dem Gemisch Wasserdampf in einer Menge von 12 kg/h zugefügt. Das Gemische verläßt das Rohr mit ca. 85°C, die Verweilzeit im Rohr beträgt ca. 10 Sekunden. Das Gemisch läuft nun in einen Fliehkraftseparator von 1 Liter Inhalt und wird dort in eine Schmelzphase und eine wäßrige Phase getrennt. Die Verweilzeit der Schmelze im Separator beträgt weniger also 60 Sekunden. Die Schmelze fließt über ein Vorratsgefäß von 0,5 l Inhalt einem Dünnschichtverdampfer von 0,2 m² Heizfläche zu, der bei 120°C und 30 Torr betrieben wird. An seinem unteren Ende werden ca. 2,5 Nm³/h Stickstoff gleicher Temperatur eingeblasen. Aus dem Sumpf des Dünnschichtverdampfers werden ca. 30 kg/h einer Schmelze von MBS abgezogen, deren Restgehalt an Morpholin ca. 0,2 Gew.-% beträgt, und die eine sehr gute Lagerstabilität besitzt.

### Beispiel 3

138 kg/h eines Reaktionsgemisches, bestehend aus 20 Gew.-% 2-Benzthiazolylsulfensäure - 2,6 - dimethylmorpholid, 20% 2,6-Dimethylmorpholin, 7,5% Natriumchlorid, 51% Wasser und 1,5% Nebenprodukten werden in gleicher Weise behandelt, wie in Beispiel 2 beschrieben. Man erhält 27,5 kg einer Schmelze von 2 - Benzthiazolylsulfensäure - 2,6 - dimethylmorpholid von sehr guter Lagerstabilität, die durch Abkühlen auf einer Schabewalze in eine handelsübliche Form überführt werden kann.

**Patentanspruch**

1. Verfahren zur Herstellung von 2-Benzthiazolylsulfensäure - morpholid durch Umsetzung von 2-Benzthiazolyldisulfid, Mercaptobenzthiazol oder dessen Natriumsalz mit Morpholin unter Einwirkung eines Oxiodationsmittels in wäßrigem Medium, dadurch gekennzeichnet, daß aus der erhaltenen Suspension, die pro Mol 2-Benzthiazolylsulfensäuremorpholid mehr als 0,5 Mol Morpholin enthält, das Reaktionsprodukt in geschmolzenem Zustand abgetrennt wird durch
a) Erhitzen der Suspension unter Aufschmelzen des festen Reaktionsproduktes,
b) anschließende schnelle Abtrennung der Schmelzphase von der wäßrigen Phase, und
c) Entfernung der in der Schmelzphase noch vorhandenen flüchtigen Anteile,
wobei die Summe der Verweilzeiten zwischen Beginn und Ende der drei Verfahrensschritte a) bis c) nicht mehr also 15 Minuten beträgt.

## Revendication

1. Procédé de fabrication de morpholide d'acide 2 - benzothiazolylsulfénique par réaction de disulfure de 2-benzothiazolyle, de mercaptobenzothiazol ou de son sel de sodium avec de la morpholine sous l'action d'un agent oxydant en milieu aqueux, caractérisé en ce qu'à partir de la suspension obtenue, qui contient par mole de morpholide d'acide 2 - benzothiazolylsulfénique plus de 0,5 mole de morpholine, on sépare le produit de réaction à l'état fondu par
a) chauffage de la suspension avec fusion du produit de réaction solide,
b) séparation consécutive rapide de la phase fondue d'avec la phase aqueuse et

c) élimination des fractions volatiles encore présentes dans la phase fondue,
la somme des temps de séjour entre le début et la fin des trois stades opératoires a) à c) ne dépassant pas 15 minutes.

## Claim

1. A process for the preparation of 2-benzothiazolylsulphenic acid morpholide by the reaction of 2-benzothiazolyl disulphide, mercaptobenzothiazole or its sodium salt with morpholine in an aqueous medium under the action of an oxidizing agent, characterised in that the reaction product is separated in the molten state from the resulting suspension, which contains more than 0.5 mol of morpholine per mol of 2-benzothiazolyl sulphenic acid morpholide, by
(a) heating the suspension with melting of the solid reaction product,
(b) subsequent rapid separation of the melt phase from the aqueous phase, and
(c) removal of volatile constituents still present in the melt phase,
the sum of residence times from beginning to end of the three stages (a) to (c) amounting to not more than 15 minutes.